# EUROPEAN PATENT APPLICATION

(11) **EP 1 738 821 A1**
(43) Date of publication of application: **03.01.2007**
(21) Application number: 05425433.9
(22) Date of filing: 17.06.2005
(51) Int. Cl.: B01J 20/22, A24D 3/14, A24B 15/30, C07C 43/225

(54) **Method of reducing the level of nitrogen oxides in a medium by absorption with resorcin¬4|arenes**

(71) Applicant: British American Tobacco Italia S.p.A., 00144 Rome (IT)
(72) Inventor: Nunziata, Alfredo, Via Amsterdam 147, 00144 Rome (IT); Lionetti, Giovanni, Via Amsterdam 147, 00144 Rome (IT); Pierri, Elena, Via Amsterdam 147, 00144 Rome (IT); Botta, Bruno c/o Dip. Studi di Chimica Technologia, P.Ie A. Moro 5, 00185 Rome (IT); Cancelliere, G. Dip. Studi di Chimica Technologia, P.Ie A. Moro 5, 00185 Rome (IT); D'Acquarica, I. Dip. Studi di Chimica Technologia, P.Ie A. Moro 5, 00185 Rome (IT); Delle Monache, G Dip. Studi di Chimica Technologia, P.Ie A. Moro 5, 00185 Rome (IT); Gasparrini, F. Dip. Studi di Chimica Technologia, P.Ie A. Moro 5, 00185 Rome (IT); Nevola, L. c/o Dip. Studi di Chimica Technologia, P.Ie A. Moro 5, 00185 Rome (IT); Subissati, D. Dip. Studi di Chimica Technologia, P.Ie A. Moro 5, 00185 Rome (IT); Villani, C. c/o Dip. Studi di Chimica Technologia, P.Ie A. Moro 5, 00185 Rome (IT)
(74) Representative: Harding, Charles Thomas

(57) **Abstract**

The present invention relates to the use of resorcin[4]arenes to absorb one or more nitrogen oxides in a nitrogen oxide-containing medium.

Further aspects of the invention relate to NO⁺ complexes with resorcin[4]arenes and filter elements comprising one or more resorcin[4]arenes.

## Description

The present invention relates to a method of reducing the level of nitrogen oxides in a medium, for example in toxic gases derived from from tobacco combustion, power plants, or large-scale industrial processes. More specifically, but not exclusively, the invention relates to a method of filtering or absorbing nitrogen oxides formed by the combustion of tobacco-containing smoking articles such as cigarettes and cigars.

### BACKGROUND TO THE INVENTION

Nitrogen dioxide (NO₂) is the major component of the so-called NOₓ gases family.^{1,2} NO_{X} is the general formula used to describe a mixture of NO₂, nitrogen monoxide (NO), and other nitrogen oxides. These are toxic gases derived, *inter alia,* from tobacco combustion, power plants, and large-scale industrial processes. NOₓ's are aggressively involved in various nitrosation processes in biological tissues. Free radical NO rapidly reacts with oxygen, producing N₂O₃ (e.g., NONO₂) and NO₂/N₂O₄. These are powerful nitrosating agents, both in the gas phase and in solution.

### STATEMENTS OF INVENTION

The present invention describes host-guest complexes formed upon interaction between NO₂ and simple synthetic macrocycles named resorc[4]arenes. The applicant has found that (a) resorc[4]arenes react with NO₂ to form stable nitrosonium (NO⁺) complexes; (b) these resorcarene-NO⁺ complexes are deeply coloured and can dissociate and bleach upon addition of water; (c) these complexes can be utilized for the NO⁺ transfer processes and nitrosation reactions. Accordingly, the invention offers a novel process of NO₂ entrapment and chemical utilization which may in turn lead towards novel, supramolecular NO₂-storing materials.

Alternatively expressed, the resorc[4]arene can act as absorbents for NO₂ to form stable nitrosonium (NO⁺) complexes. Thus, the resorc[4]arene is sometimes referred to as an absorbent of the invention.

A first aspect of the invention relates to the use of a resorcin[4]arene to absorb one or more nitrogen oxides in a nitrogen oxide-containing medium.

A second aspect of the invention relates to a method of forming a nitrosonium complex, said method comprising contacting a resorc[4]arene with a nitrogen oxide.

A third aspect of the invention relates to nitrosonium complex of formula II 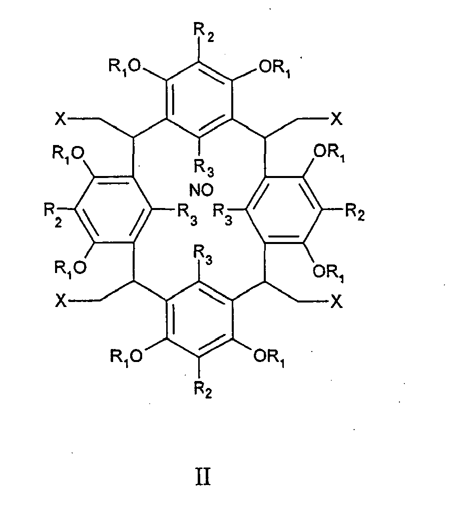 wherein
each R₂ and R₃ is independently H, alkoxy, alkyl, aryl, aralkyl, halogen, cyano, nitro or carboxylate;
each X is independently COOR₄ or CH₂-halogen;
each R₄ is independently H, alkyl, aryl, aralkyl or -(CH₂)ₙCH₂Y;
each n is independently 0 to 20;
each Y is independently CH₂-halogen or CH=CR₅R₆;
each R₁, R₅ and R₆ is independently H, alkyl, aryl or aralkyl; wherein said alkyl, aryl and aralkyl groups may be optionally substituted by one or more substituents selected from halogen, OH, alkoxy, NO₂, NH₂, N(alkyl)₂ (preferably NMe₂), C(halo)₃ (preferably CF₃), carboxylate and cyano.

Thus, a preferred third aspect of the invention relates to nitrosonium complex of formula IIx 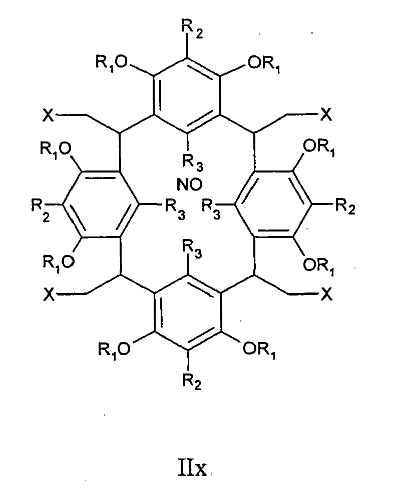 wherein
each R₂ and R₃ is independently H, alkoxy, alkyl, aryl, aralkyl, halogen, cyano, nitro or carboxylate;
each X is independently COOR₄ or CH₂-halogen;
each R₄ is independently H, alkyl, aryl, aralkyl or -(CH₂)ₙCH₂Y;
each n is independently 0 to 20;
each Y is independently CH₂-halogen or CH=CR₅R₆;
each R₁, R₅ and R₆ is independently H, alkyl, aryl or aralkyl; wherein said alkyl, aryl and aralkyl groups may be optionally substituted by one or more substituents selected from halogen, OH, alkoxy, NO₂, NH₂, NMe₂, CF₃, carboxylate and cyano.

A fourth aspect of the invention relates to a complex obtained by contacting a resorc[4]arene with a nitrogen oxide.

A fifth aspect of the invention relates to the use of a complex according to the invention as a nitrosating reagent or as an NO⁺ transfer agent.

A sixth aspect of the invention relates to a method of entrapping one or more nitrogen oxides, said method comprising contacting one or more resorc[4]arenes with a gaseous mixture comprising one or more nitrogen oxides.

A seventh aspect of the invention relates to a method of absorbing one or more nitrogen oxides from tobacco smoke, said method comprising filtering the tobacco smoke through a filter element comprising one or more resorc[4]arenes.

An eighth aspect of the invention relates to a method of selectively absorbing nitrogen oxide-containing combustion products from tobacco smoke, said method comprising allowing tobacco smoke to come into contact with a filter element comprising one or more resorc[4]arenes.

A ninth aspect of the invention relates to a filter element for absorbing one or more nitrogen oxides in a gaseous mixture, said filter element comprising one or more resorc[4]arenes and optionally one or more additional filter materials.

A tenth aspect of the invention relates to a filter element for selectively absorbing NO₂₋containing combustion products produced by burning a smoking article, said filter element comprising as filter material one or more resorc[4]arenes.

An eleventh aspect of the invention relates to a smoking article comprising a filter element according to the invention.

A twelfth aspect of the invention relates to a filtration apparatus for absorbing, or reducing the level of, nitrogen oxides in a gaseous mixture, said apparatus comprising:
a chamber for receiving the gaseous mixture;
inlet and/or outlet means;
means for absorbing, or reducing the level of, one or more nitrogen oxides, said
means comprising one or more resorc[4]arenes; and
optionally a fluid transfer means for circulating the gaseous mixture around the chamber.

Other aspects of the invention include a smoking article or a component thereof comprising smoking material and an absorbent of the invention ― i.e. one or more of the resorc[4]arenes referred to herein. The component of the smoking article incorporating the absorbent may be a smoke filter, a wrapper or casing for the smoking article, filter material such as filter tow, or the smoking material itself. The invention specifically includes a smoke filter comprising an absorbent of the invention ― i.e. one or more of the resorc[4]arenes referred to herein.

The smoking article of the invention may take any form. For example the smoking article may be one in which the tobacco is smoked by igniting the smoking material and inhaling the products of combustion, as for example in a cigarette, cigar or cigarillo. Alternatively the smoking article may be one in which the smoking material is heated to a temperature at which decomposition in to pyrolysis products occurs without combustion. Such articles are well known and incorporate electrical or other heating means such as a charcoal element.

In particular the smoking article may comprise a rod of smoking material optionally in a casing, with or without a filter. The casing may be a wrapper of paper, tobacco leaf or reconstituted tobacco. Alternatively, where, for example, the smoking article is intended to produce low emissions of sidestream smoke, or lower levels of pyrolysis products in the mainstream smoke, the casing may be composed of non-combustible inorganic material such as a ceramic material. The filter may be of any suitable material, for example fibrous cellulose acetate, polypropylene or polyethylene, or paper.

The smoking material is preferably tobacco but may be a non-tobacco smoking material. Examples of non-tobacco smoking materials are dried and cured vegetable material, including fruit materials, and a synthetic smoking material such as may be produced from alginates and an aerosol-generating substance such as ethylene glycol.

The smoking material may comprise a blend of tobacco and non-tobacco smoking materials. Where the smoking material comprises tobacco, the tobacco may of any suitable type, or a blend thereof, including air-cured, fire-cured, flue-cured, or suncured lamina or stem, and may have been processed using any appropriate process. For example, the tobacco may be cut, shredded, expanded or reconstituted. The smoking material may also include conventional additives, such as ameliorants, colorants, humectants (such as glycerol and propylene glycol), and flavourings (such as sugar, liquorice and cocoa).

The absorbent may be incorporated in the smoking material. Accordingly, the invention includes smoking material containing an absorbent of the invention ― i.e. one or more of the resorc[4]arenes referred to herein.

Alternatively, where the smoking article comprises a rod of smoking material in a wrapper, the absorbent may be incorporated in the wrapper. The invention therefore includes wrapper material for smoking articles containing an absorbent of the invention ― i.e. one or more of the resorc[4]arenes referred to herein. The wrapper may be a cellulose-based material such a paper, or a tobacco based material such as reconstituted tobacco.

As a further alternative, if the smoking article comprises a filter, the absorbent may be incorporated in the filter.

The present invention also includes a smoke filter for a smoking article comprising an absorbent of the invention ― i.e. one or more of the resorc[4]arenes referred to herein. The smoke filter may be produced separately from the smoking article, for example in the form of a cigarette or cigar holder, or it may be integrated into the smoking article, for example in the form of a cigarette with a filter tip.

Smoke filters in the form of filter tips may be of any conventional construction. For example it may in the form of a "dalmatian" filter comprising a section of fibrous filter material, such a cellulose acetate, the absorbent of the invention being in particulate form and distributed throughout the section. Alternatively the filter may be in the form of a "cavity" filter, comprising multiple sections, the absorbent of the invention being confined to one such section. For example the porous carbon material may lie between two adjacent section of fibrous filter material.

The smoke filter may also comprise other adsorbent materials. The absorbent of the invention may also be mixed with other adsorbent materials in a cavity or dalmatian type filter element.

The preferred smoking articles of the invention are cigarettes, comprising a rod of tobacco, wrapper, and a smoke filter, the absorbent of the invention being incorporated in the smoke filter.

### DETAILED DESCRIPTION

As used herein, the term "nitrogen oxide" encompasses species having the general formula NOₓ, and which include NO₂, nitrogen monoxide (NO), and other nitrogen oxides.

As mentioned above, the present invention relates to the use of macrocyclic species known as resorc[4]arenes.

As used herein, the term "resorc[4]arene" is used synonymously with "resorcinarene". The present claims encompass the use of a single resorcin[4]arene, or combinations of one or more resorcin[4]arenes.

As used herein, the term "resorc[4]arene" refers to a macrocycle formed from four phenyl groups linked together by four methylene bridges (each methylene being optionally substituted), each phenyl group bearing two OR' substituents as shown. The core structure of a resorcinarene is as follows:

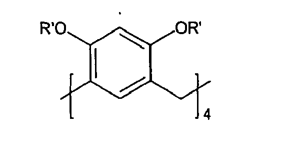

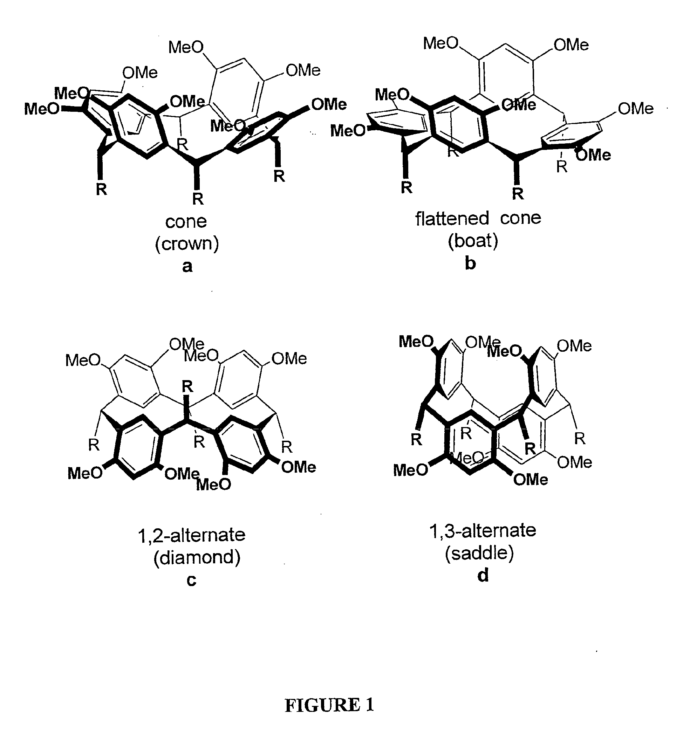

Resorc[4]arenes are conformationally flexible and can adopt a 1,2-alternate, 1,3-alternate or flattened cone conformation (see Figure 1). The molecules are popular building blocks in molecular recognition for the construction of nanoscale containers such as cavitands, carcerands, and capsules.³ Cone-shaped resorc[4]arenes are ~ 4 Å deep and ~ 7 Å in diameter at the upper rim. The cone conformation in solution results from the equilibrium of two pinched conformations (C*₂ᵥ* symmetry), where two opposite aromatic rings are alternatively almost parallel and at ~ 5 Å distance, while the two others are flattened. At room temperature, the interconversion of the two C*₂ᵥ* structures is fast on the NMR time scale and, as a result, a simple spectrum corresponding to a C*₄ᵥ* symmetry is obtained.⁴ Resorc[4]arenes in the 1,3-alternate conformation (Figure 1) are much more rigid and possess a quasi-cylindrical inner tunnel, defined by two co-facial pairs of aromatic rings orthogonally oriented along the cavity axis. Conversely, in the 1,2-alternate conformation, two neighbour rings point to one side and the two others to the opposite; while the substituents are all axial in the previous two forms, the 1,2-alternate conformation is characterized by the presence of an equatorial substituent.

Complexes of resorc[4]arenes with neutral molecules are generally weak, because the cavities itself are too small and also lack additional binding sites. In most of the crystal structures of inclusion complexes of resorc[4]arenes, the guest molecule is positioned not inside but roughly above the plane defined by the upper carbon atoms of the cyclic polyaromatic skeleton. On the other hand, cations are known to more strongly interact with the resorcarene π-surface. Ammonium ions and metal cations were shown to be complexed within the cone-shaped cavities. 1,3-Alternate calixarenes, quite similar macrocycles to resorc[4]arenes, when functionalised with appropriate binding sites on the phenol oxygens, bind metal cations Na⁺, K⁺, and Ag⁺ both with "hard" oxygens and "soft" π-basic aromatic rings.⁵ Recently, the formation of complexes between calixarenes, and nitrosonium (NO⁺) cation, both in solution and in the solid state, has been reported.⁶ The NO⁺ cation was found encapsulated within the calixarene cavity (X-ray analysis), and strong charge-transfer interactions with the π-surface of calixarene positioned the guest between the co-facial aromatic rings at a distance 2.4 Å, which is much shorter than the typical van der Waals contact (3.2 Å).

To date, however, there has been no disclosure in the art of resorcin[4]arene complexes with NO⁺.

The unpaired electron on the nitrogen confers to NO₂ its paramagnetic properties, an intense brown-orange colour and the possibility of reacting with itself, to form a colourless dimer, dinitrogen tetroxide (N₂O₄).⁷ The N-N bond in N₂O₄ is quite weak and rapidly dissociates as the temperature is raised to give NO₂. The position of the equilibrium between the two compounds and the colour of the system vary with temperature. Below ― 21 °C, only pure, solid N₂O₄ is present, whereas above 140 °C the system is 100 % NO₂. The dynamic interconversion between NO₂ and N₂O₄ makes it impossible to study either of these species alone. N₂O₄ may disproportionate to ionic NO⁺NO3⁻ upon reacting with aromatic compounds.^{7,8}

The present invention centres on the ability of resorc[4]arenes to encapsulate NO₂/N₂O₄ gas and form stable resorc[4]arene-nitrosonium complexes.

As mentioned above, one aspect of the invention relates to a method of absorbing, or reducing the level of, one or more nitrogen oxides in a gaseous mixture, said method comprising contacting the gaseous mixture with one or more resorc[4]arenes.

In one preferred embodiment of the invention, the resorc[4]arene is of formula I 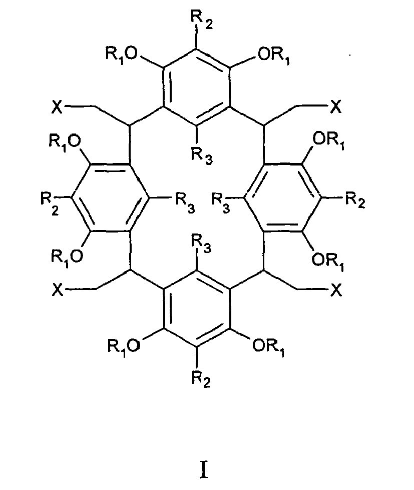 wherein
each R₂ and R₃ is independently H, alkoxy, alkyl, aryl, aralkyl, halogen, cyano, nitro or carboxylate;
each X is independently COOR₄ or CH₂-halogen;
each R₄ is independently H, alkyl, aryl, aralkyl or -(CH₂)ₙCH₂Y;
each n is independently 0 to 20;
each Y is independently CH₂-halogen or CH=CR₅R₆;
each R₁, R₅ and R₆ is independently H, alkyl, aryl or aralkyl; wherein said alkyl, aryl and aralkyl groups may be optionally substituted by one or more substituents selected from halogen, OH, alkoxy, NO₂, NH₂, N(alkyl)₂ (preferably NMe₂), C(halo)₃ (preferably CF₃), carboxylate and cyano.

As used herein, the term "alkyl" includes both saturated straight chain and branched alkyl groups which may be substituted (mono- or poly-) or unsubstituted. Preferably, the alkyl group is a C₁₋₂₀ alkyl group, more preferably a C₁₋₁₅, more preferably still a C₁₋₁₂ alkyl group, more preferably still, a C₁₋₆ alkyl group, more preferably a C₁₋₃ alkyl group. Particularly preferred alkyl groups include, for example, methyl, ethyl, propyl, isopropyl, butyl, isobutyl, tert-butyl, pentyl and hexyl. Preferably, the alkyl group is unsubstituted.

As used herein, the term "aryl" refers to a substituted (mono- or poly-) or unsubstituted monoaromatic or polyaromatic system, wherein said polyaromatic system may be fused or unfused. Preferably, the term "aryl" is includes groups having from 6 to 10 carbon atoms, e.g. phenyl, naphthyl etc. The term "aryl" is synonymous with the term "aromatic". Preferably, the aryl group is unsubstituted.

The term "aralkyl" is used as a conjunction of the terms alkyl and aryl as given above. Preferred aralkyl groups include CH₂Ph and CH₂CH₂Ph and the like.

Thus, in a more preferred embodiment of the invention, the resorc[4]arene is of formula Ix 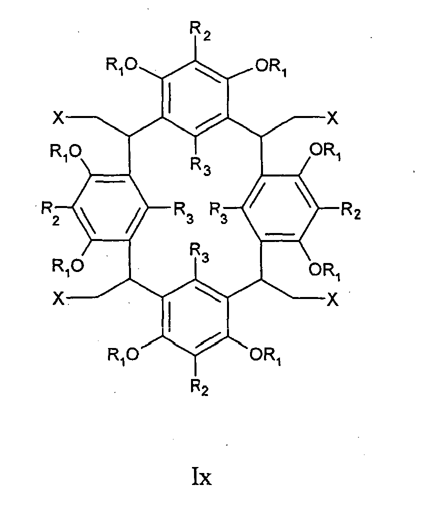 wherein
each R₂ and R₃ is independently H, alkoxy, alkyl, aryl, aralkyl, halogen, cyano, nitro or carboxylate;
each X is independently COOR₄ or CH₂-halogen;
each R₄ is independently H, alkyl, aryl, aralkyl or -(CH₂)ₙCH₂Y;
each n is independently 0 to 20;
each Y is independently CH₂-halogen or CH=CR₅R₆;
each R₁, R₅ and R₆ is independently H, alkyl, aryl or aralkyl; wherein said alkyl, aryl and aralkyl groups may be optionally substituted by one or more substituents selected from halogen, OH, alkoxy, NO₂, NH₂, NMe₂, CF₃, carboxylate and cyano.

Preferably, the halogen is Cl, Br, or I.

In one preferred embodiment, each R₂ and R₃ is H.

In one preferred embodiment, each R₁, R₅ and R₆ is independently H or alkyl.

In one preferred embodiment, each R₄ is independently H, alkyl, or -(CH₂)ₙCH₂Y;

In one preferred embodiment, each X is the same.

In one preferred embodiment, each R₁ is the same. More preferably still, each R₁ is alkyl.

In one particularly preferred embodiment,
X is COOR₄ or CH₂Br;
R₄ is H, Me, Et, ⁱPr, or -(CH₂)ₙCH₂Y;
n is 2 to 15;
Y is CH₂Br or CH=CH₂; and
R₁ is Me.

In one preferred embodiment, n is 2 to 18, more preferably 2 to 15, more preferably still 2 to 12, and even more preferably 6 to 12.

In one especially preferred embodiment,
X is COOR₄ or CH₂Br; and
R₄ is H, Me, Et, ⁱPr or -(CH₂)ₙCH₂Br;
n is 2 to 11; and
R₁ is Me.

In an alternative preferred embodiment,
X is COOR₄ or CH₂Br; and
R₄ is H, Me, Et, ⁱPr, or -(CH₂)ₙCH=CH₂;
n is 2 to 9; and
R₁ is Me.

In another preferred embodiment,
X is COOR₄ or CH₂Br; and
R₄ is H, Me, Et, ⁱPr, -(CH₂)₁₁CH₂Br or -(CH₂)₉CH=CH₂; and
R₁ is Me.

In one preferred embodiment, the medium is a gaseous medium, i.e. the medium is a gaseous mixture comprising one or more nitrogen oxides. In an alternative preferred embodiment, the medium is a liquid, i.e. a solution containing one or more nitrogen oxides in dissolved or gaseous form.

Thus, in one embodiment, the invention relates to a method of absorbing, or reducing the level of, one or more nitrogen oxides in a gaseous mixture, said method comprising contacting the gaseous mixture with one or more resorcin[4]arenes.

In one preferred embodiment, the gaseous mixture and one or more resorcin[4]arenes come into contact by means of passive diffusion.

In another preferred embodiment, the gaseous mixture is brought into contact with the one or more resorcin[4]arenes by virtue of the gaseous mixture flowing through a filter element comprising one or more resorcin[4]arenes. For example, this may occur when a gaseous mixture formed from the combustion of a smoking article is inhaled by the user through a filter element.

In one preferred embodiment, the method reduces the level of NO₂ and/or N₂O₄.

In a preferred embodiment, the method of the invention absorbs substantially all the NO₂ from the medium.

In another preferred embodiment, the method of the invention selectively absorbs NO₂ from the medium.

As used herein, the term "selectively" means that the resorcinarenes of the invention absorb a portion of, substantially all, or all, of the NO₂ in the medium in preference to other species that may be present. Where the medium is a gaseous medium, preferably, the resorcinarenes of the invention absorb a portion of, substantially all, or all, of the NO₂ in a gaseous mixture, but do not absorb other gases to any significant extent. More preferably, the resorcinarenes of the invention exhibit a selectivity ratio for NO₂ over other gases of at least 5:1, more preferably 10:1, more preferably still 50: 1, more preferably still 100:1, more preferably still 200 or 500:1.

Throughout the specification, the term "absorb/absorbing" reflects the ability of the resorcin[4]arenes of the invention to form complexes with the nitronium ion, NO⁺.

### SURFACE LINKED RESORC[4]ARENES

For potential applications in filter preparation technologies, receptor molecules are preferably linked to solid supports or surfaces. Although a wide variety of polymeric supports are now commercially available, NO₂ (and NO_{X} oxides in general) easily react with many of them, causing destruction and aging.⁷ As a free radical, NO₂ readily attacks double bonds in polybutadienes, polyisoprenes and their copolymers, ester groups in poly(methyl)methacrylate, and also amide fragments in polyamides and polyuretanes. Furthermore, NO⁺ can be generated from various NO_{X} oxides, and react with alkenes and other double bond containing structures.⁸

In one particularly preferred embodiment of the invention, the resorc[4]arene is tethered or linked to a support material, for example a solid support or surface.

Preferred support materials include, for example, modified or functionalised silica gel. Preferably, the resorc[4]arene is attached to the solid support or surface by one or more covalent bonds.

In one especially preferred embodiment the support material is aminopropylated silica gel. Aminopropylated silica gel may be prepared by treating silica gel with 3-aminopropyltriethoxysilane under the appropriate conditions.

Preferably, the resorc[4]arene is linked to the aminopropylated silica gel via a bond formed by reacting the amino group of aminopropylated silica gel with a resorc[4]arene of formula Ia, 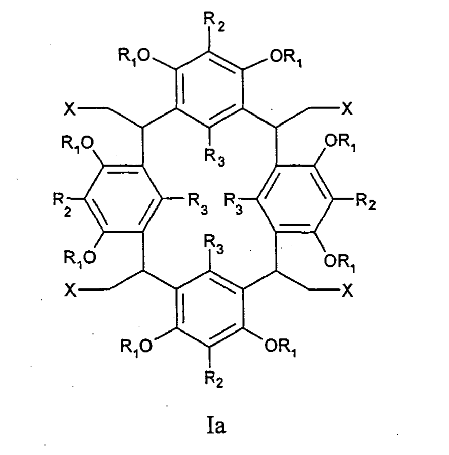 wherein
each R₂ and R₃ is independently H, alkoxy, alkyl, aryl, aralkyl, halogen, cyano, nitro or carboxylate;
each X is independently COOR₄ and each R₄ is -(CH₂)ₙCH₂Y;
each n is independently 0 to 20;
each Y is independently CH₂-halogen;
each R₁ is independently H, alkyl, aryl or aralkyl;
wherein said alkyl, aryl and aralkyl groups may be optionally substituted by one or more substituents selected from halogen, OH, alkoxy, NO₂, NH₂, NMe₂, CF₃, carboxylate and cyano.

Preferably, for said compound of formula Ia:
X is COOR₄ and R₄ is -(CH₂)ₙCH₂Y;
n is 0 to 20;
Y is CH₂-halogen, wherein halogen is Cl, Br, or I;
R₁ is alkyl; and
R₂ and R₃ are H;
R₁ is alkyl.

For this embodiment of the invention, preferably the halogen is Br.

As used herein, the term "resorcinarene" or "resorcin[4]arene" encompasses both the compounds *per se,* and the compounds when attached to a solid support material.

### NITROSONIUM COMPLEX

A further aspect of the invention relates to a complex of a resorc[4]arene and a nitrosonium ion, NO⁺. Such complexes may be formed by contacting the resorc[4]arene with a nitrogen oxide, for example, NO₂.

A preferred embodiment of the invention relates to nitrosonium complexes of formula II 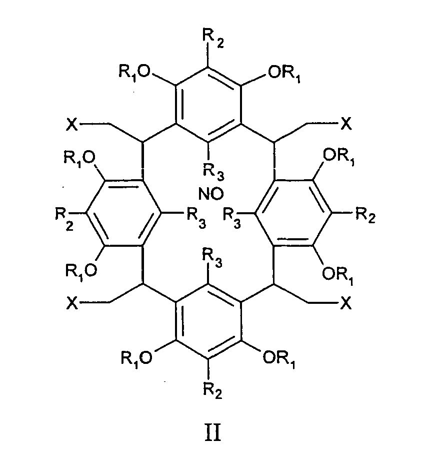 wherein R₁, R₂, R₃ and X are as defined above for compounds of formula I.

Nitrosonium complexes according to the invention are useful as nitrosating reagents and/or as NO⁺ transfer agents in chemical processes.

### TOBACCO PRODUCTS

In one embodiment of the invention, the method is used to reduce the level of, or remove, one or more nitrogen oxides in gaseous mixtures produced by burning tobacco.

A further aspect of the invention relates to a filter element for absorbing one or more nitrogen oxides in a gaseous mixture, said filter element comprising one or more resorc[4]arenes and optionally one or more additional filter materials.

Preferably, the filter element is for use with a smoking article.

It is well established in the art that the concentration of undesirable substances in the main smoke stream generated by smoking articles, particularly cigarettes, can be reduced with the aid of filters at the mouth end of the smoking article. A variety of different filter materials and filter constructions are known in the art and will be familiar to the skilled artisan.

Another aspect of the invention relates to a filter element for selectively absorbing NO₂-containing combustion products produced by burning a smoking article, said filter element comprising as filter material one or more resorc[4]arenes, and optionally one or more additional filter materials.

Preferably, the additional filter material is a fibrous material. The prime purpose of such fibrous filter materials is to remove part of the aerosol particles, "tar", from the smoke.

In a preferred embodiment, the additional filter material is a polymer, preferably having a large surface area, such as branched polymer. Preferred polymers include fibrous cellulose-based material, partially oxidized cellulose, polyaniline, regenerated cellulose, cellulose acetate and the like. Where the additional filter material is a polymer, suitable examples include linear, branched or dendritic polymers, or combinations or mixtures thereof. The polymer may be a natural polymer, or a derivative thereof, or a synthetic polymer, or mixtures or copolymers thereof. Partially oxidized cellulose is an example of a natural polymer useful as an additional filter material. Other polymers include those based on aniline and derivatives thereof.

In an alternative preferred embodiment, the additional filter material may be an inorganic oxide, preferably a zeolite. Suitable inorganic oxides include, for example, natural zeolite, synthetic zeolite, or mixtures or derivatives thereof. Inorganic oxides may be crystalline, amorphous, or mixtures thereof. Preferred inorganic oxides include oxides of aluminum, silicon, and combinations thereof.

In yet another preferred embodiment, the additional filter material is an activated carbon.

In another preferred embodiment, the additional filter material is a combination of two or more of the above-mentioned materials. For instance, the different materials may be simply mixed together and the mixture applied to a filter space comprised of two sections of particle-filter with an intermediate chamber at the mouth end of a cigarette. Alternatively in another embodiment, the filter may be divided into several compartments which may contain different filter materials or different mixtures of filter materials.

The purpose of the additional filter material is to create interaction between the main smoke stream and the filter material so that a mass flow exists through the filter element combined with a high surface area for providing sufficient interaction between the gas phase main smoke stream and the resorcinarenes of the invention. For example, inorganic oxides are selected for their developed pore structure which allows the combustion products of the tobacco be drawn through the filter element at an acceptable flow rate to maintain the organoleptic pleasure of a smoker.

The filter material may optionally contain further components, for example synthetic polymers such as polyolefins (e.g. polyethylene, polypropylene, etc.), poly(vinyl alcohol), polyesters (e.g. polyethylene terephthalate) and polyamides; natural celluloses derived from wood fibers (e.g. soft wood pulp, hard wood pulp, etc.), seed fibers (cotton such as linter, bombax cotton, kapok, etc.), bast fibers (e.g. hemp, linen, jute, ramie, paper mulberry, paper bush (mitsumata), etc.), and leaf fibers (e.g. Manila hemp, New Zealand flax, etc.); and regenerated celluloses such as viscose rayon, cuprammonium rayon, Fortisan, nitrate rayon, etc. Natural cellulose (especially wood pulp and linter pulp) and regenerated cellulose are particularly preferred.

The filter materials may also contain one or more additives such as sizing agents e.g. finely divided inorganic substances such as powders of kaolin, talc, diatomaceous earth, titanium dioxide, alumina, quartz, calcium carbonate, barium sulfate, etc.; thermal stabilizers such as salts of the alkali metals and alkaline earth metals; colorants; yield improvers; biodegradation accelerators such as citric acid, tartaric acid, malic acid or the like and/or photodegradation accelerators such as anatase titanium dioxide.

The filter material may also contain a plasticizer such as triacetin or triethylene glycol diacetate and/or a water soluble adhesive. Suitable water soluble adhesices include for example, natural adhesives (e.g. starch, modified starch, solubilized starch, dextran, gum arabic, sodium alginate, casein, gelatin, etc.); cellulose derivatives (e.g. carboxymethylcellulose, hydroxyethylcellulose, methylcellulose, ethylcellulose, etc.), synthetic resin adhesives (e.g. poly(vinyl alcohol), polyvinylpyrrolidone, poly(vinyl ether), water-soluble acrylic resin, poly(vinyl acetate), vinyl alkyl ether-maleic acid copolymer, poly(alkylene oxide), water-soluble polyester, water-soluble polyamides, and the like.

Another aspect of the invention relates to a smoking article comprising a filter element according to the invention.

Preferably, the smoking article is a cigarette, cigar, cigar holder or pipe.

In one preferred embodiment, the filter element is a tip added to a smoking article prior to the smoking of the smoking article. The tip may be added by a smoker.

In one particularly preferred embodiment of the invention, at least one additional filter element may be provided to filter other combustion products. Preferably, this additional filter element is a cellulose acetate filter element. This additional filter element may be incorporated into the filter tip or it may be incorporated into a holder for the smoking article ― such as a cigarette holder.

### OTHER APPLICATIONS

The present invention is not limited to applications in the tobacco industry. Indeed, the presently claimed invention is applicable to any medium in which it is desirable to absorb, remove, or reduce the level of, nitrogen oxides. By way of example, other applications may include any industrial process that produces toxic gases, for example large scale plants, power production, etc.

Thus, the above-described filter element need not necessarily be limited to use in, or with, smoking articles.

An alternative aspect of the invention therefore relates to a filtration apparatus for absorbing, or reducing the level of, one or more nitrogen oxides in a gaseous mixture, said apparatus comprising:
a chamber for receiving the gaseous mixture;
inlet and/or outlet means;
means for absorbing, or reducing the level of, one or more nitrogen oxides, said
means comprising one or more resorc[4]arenes; and
optionally a fluid transfer means for circulating the gaseous mixture around the chamber.

Preferably, the fluid transfer means is a pump.

In one preferred embodiment of the invention, the chamber is a closed system.

The present invention is further described by way of the following examples, and with reference to the accompanying figures, wherein:
Figure 1 shows the four different conformations that resorc[4]arenes can adopt. These are (a) cone (crown); (b) flattened cone (boat); (c) 1,2-alternate (diamond); and (d) 1,3-alternate (saddle).
Figure 2 shows the structure of a resorc[4]arenes with the four aryl groups labelled A-D and the carbons labelled 1-28. The NMR characterisation of the complex is discussed in more detail in the accompanying examples.
Figure 3 shows formation of a nitrosonium complex with compound 7. NMR studies suggest that the NO⁺ guest lies next to one of the two rings (7 is shown in the flattened-cone conformation, C*₂ᵥ* group of symmetry).
Figure 4 shows how resorcarene **10** can be readily reacted with aminopropyl functionalized silica-gel, giving the immobilized system **10a.**
Figure 5 is a side elevation, partly in longitudinal cross-section and partially broken away of a smoking article with a smoke filter according to the invention.
Figure 6 is a similar view to Figure 5 of a smoking article with an alternative smoke filter according to the invention.

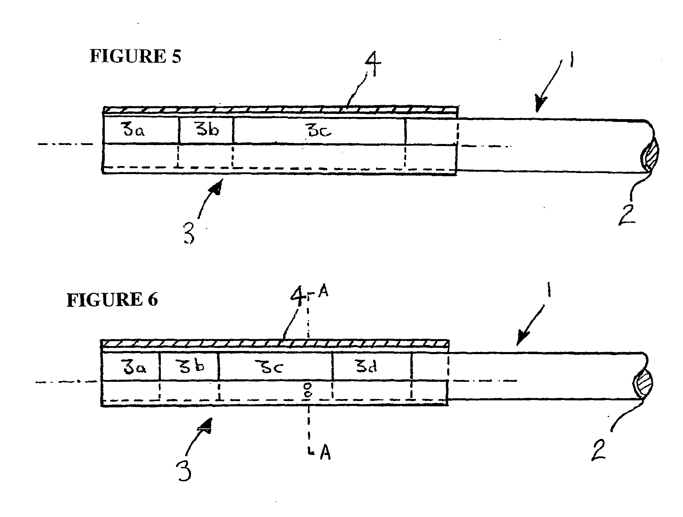

In Figures 5 and 6, similar features are given like reference numerals.

### EXAMPLES

### Synthesis of resorc[4]arenes

Resorc[4]arenes tetraesters **1-3** (Scheme 1) in the cone, 1,3-alternate and 1,2-alternate conformations, respectively, were synthesized starting from (*E*)-2,4-dimethoxycinnamic acid ethyl ester and using BF₃·Et₂O as catalyst.¹⁰ Reduction¹¹ of tetraesters **1-3** yielded tetraalcohols **4-6,** while the following bromination of **4-6** gave the tetrabromides **7-9.**

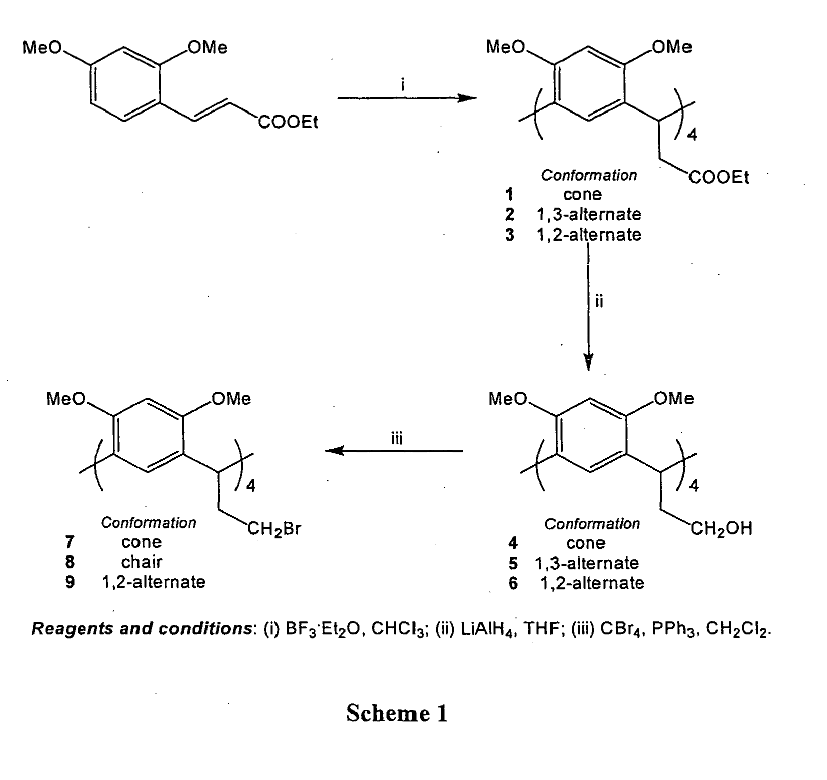

Compound **11** was obtained by BF₃·Et₂O cyclization of (*E*)-2,4-dimethoxy-cinnamic acid-11-bromoundecyl ester (Scheme 2).

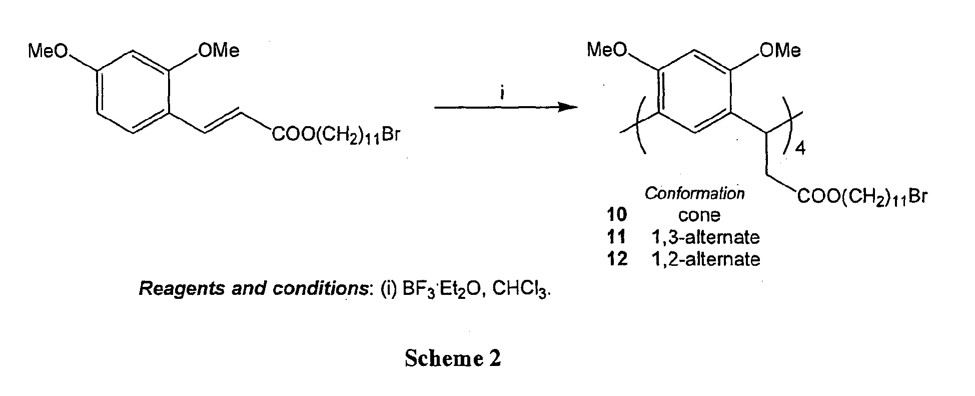

### Interaction of resorc[4]arenes with NO₂

Treatment of CHCl₃ solutions of **1, 2, 7, 9** and **11** with bubbling NO₂ resulted in a deep coloration at once: the solutions turned blue **(1),** initially violet and deep blue after a week **(2),** deep purple **(7** and **9).** The UV-vis spectra of the NO₂-exposed solutions changed as summarized in Table 1: in all case two broad absorption bands appeared at longer wavelength, suggesting the possibility of the NO⁺ coordination inside by a charge-transfer mechanism. By contrast, in blank experiments performed with the monomer (*E*)-2,4-dimethoxycinnamic acid ethyl ester, only a pale coloration was observed upon exposure to NO₂. This finding emphasizes the importance of the cavity for the NO₂ entrapment.

These complexes are very stable and can be stored, in the absence of moisture, for several weeks, both in CHCl₃ solution and in the solid state.

**Table 1. UV-vis spectra of free hosts and their NO⁺ complexes.**

| **Host** | **[H]** | **[λ]_{H}** | **Ratio [H]/[G]** | **[λ]_{H+G}** |
|---|---|---|---|---|
| **1** | 10-² M | 275 nm | 1/2 | 375 nm; 630nm |
| **2** | 10-² M | 275 nm | 1/5 | 375 nm; 580 nm |
| **7** | 10⁻⁴ M | 280 nm | 1/10 | 334 nm; 622 nm |
| **9** | 10⁻² M | 280 nm | 1/5 | 373 nm; 630 nm |
| **11** | 10⁻² M | 275 nm | 1/5 | 375 nm |

As the instant coloration implied charge-transfer between the prereactive nitrosating/nitrating species^{2,7,8} and **1, 2, 7,** and **9,** such implication was monitored also by ¹H NMR analysis of the solutions of the complexes.

### Analysis and characterization of NO⁺ complexes

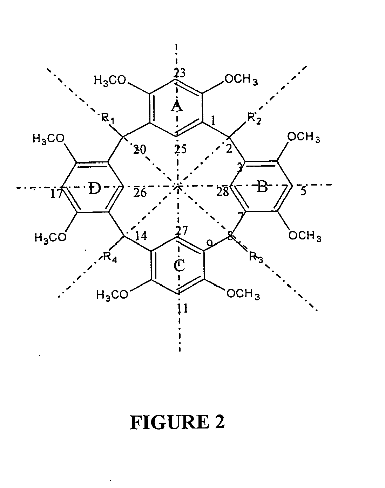

Table 2 shows selected ¹H NMR spectral data of the free hosts **1, 2, 7, 9, 11** and of the corresponding NO⁺ complexes. The NO⁺ exchange in and out of the cavity is slow on the NMR time scale and separate signals for both free and complexed species can be observed. In particular, the two He aromatic protons (at 6.46 ppm) and the two Hᵢ aromatic protons (at 6.86 ppm) of the free host 2 (1,3-alternate conformation) are shifted downfield, to 6.72 and 7.39 ppm, respectively, in the nitrosonium complex. The same happens to one (12H, s, at 3.65 ppm) of the methoxyl groups signals, shifted to 3.71 ppm. These results can be attributed to the presence of entrapped NO⁺ guest in the upper rim of the resorc[4]arene, affecting the neighbour protons (e.g. 5 and 17 He protons, their *ortho*-methoxyl groups and 25 and 27 Hᵢ protons; see Figure 2). Very likely, the NO⁺ guest is released by one aromatic ring (more precisely by its upper part) to be captured by the opposite aromatic ring in a process fast for the NMR time scale.

**Table 2. NMR spectral data of free hosts and their NO⁺ complexes.**

| Host | Proton position | Free host | Host·NO⁺ complexes |
|---|---|---|---|
| | 2, 14; 8, 20 | 4.96 (t) | 4.89 (m) |
| **1** | 5, 17; 11,23 | 6.31 (s) | 6.32 (s); **6.44** (2H,m); **6.85** (s) |
| *cone* | 25, 27; 26, 28 | 6.52 (s) | 6.53 (s); **6.85** (2H, d); **7.85** (s) |
| | OMe | 3.64 (s) | 3.75 (m); **3.84** (6H,m); **3.88** (s) |
| | 2, 14; 8, 20 | 4.63 (t) | 4.70 (t); 4.75 (m) |
| **7** | 5, 17; 11, 23 | 6.32 (s) | **6.40** (s); **6.45** (s) |
| *cone* | 25, 27; 26, 28 | 6.56 (s) | 6.49 (s); 6.54 (s) |
| | OMe | 3.62 (s) | **3.72** (s); **3.84** (s) |
| | 2, 14; 8, 20 | 5.04 (dd) | 4.98 (dd) |
| **2** | 5, 17; 11, 23 | 6.46 (s); 6.40 (s) | **6.72** (s); 6.40 (s) |
| *1,3-alternate* | 25, 27; 26, 28 | 6.82 (s); 6.22 (s) | **7.39** (s); 6.21 (s) |
| | OMe | 3.85 (s); 3.68 (s) | 3.88 (s); **3.71** (s) |
| | 2, 14; 8, 20 | 4.97 (m); 5.80 (m) | 4.97 (m); 5.81 (m) |
| **11** | 5, 17; 11, 23 | 6.46 (s); 6.39 (s) | **6.75(s);** 6.445 (s) |
| *1,3-alternate* | 25, 27; 26, 28 | 6.81 (s); 6.18 (s) | **7.32** (s); 6.20 (s) |
| | OMe | 3.88 (s); 3.63 (s) | 3.86 (s); **3.75** (s) |
| | 2; 14; 8, 20 | 5.22 (t); 4.74 (m); | **5.30** (t); 4.55 (m); 4.68 (m) |
| | 5, 23; 11, 17 | 4.77 (m) | **8.78** (s); 6.43 (m) |
| **9** | 26, 27; 25, 28 | 6.43 (s); 6.39 (s) | **9.75** (s); 6.62 (s) |
| *1,2-alternate* | 4,24 ; 6,22 (OMe) | 7.38 (s); 6.47 (s) | **4.10** (s) ; **3.86** (s) |
| | 10,18;12,16 (OMe) | 3.88 (s); 3.68 (s) | 3.77 (s) ; 3.85 (s) |
| | | 3.78 (s); 3.86 (s) | |

Similar considerations are valid for compound **11** with the same conformation.

In the ¹H NMR spectrum of the complex 7·NO⁺SbF₆⁻, all the proton signals of the free **7** (cone conformation, C₄ᵥ symmetry) are doubled. For instance, the Hᵢ aromatic protons (4H, s, at 6.56 ppm in the free 7) are shifted to two singlets (6.49 and 6.54 ppm, respectively, 2H each) in the complex. These results suggested that host 7 lost its C₄ᵥ symmetry for a C₂ᵥ one after the NO⁺ entrapment. This can be interpreted as a freezing of one of the two flattened-cone forms of the equilibrium, with the guest oscillating between the two face-to-face rings.

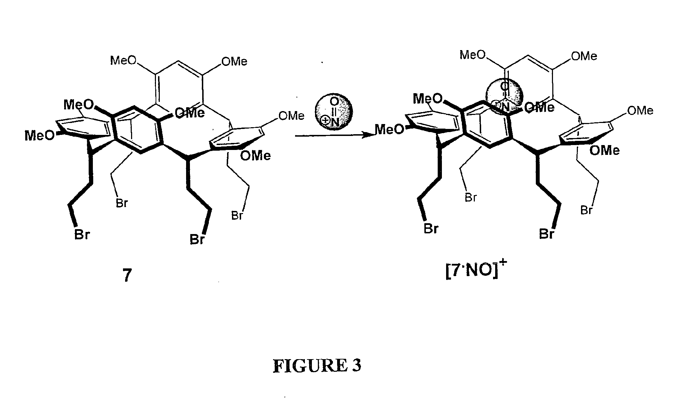

In the ¹H NMR spectrum of the nitrosonium complex with resorcarene **1,** the original signals of the free form are shifted to a more complex distribution pattern. The He aromatic protons (4H at 6.31 ppm in the spectrum of the free host 1), for instance, give three signals at 6.32, 6.44 and 6.53 ppm, integrated as 1:2:1 respectively. Analogously, the signals for the methoxyl groups and Hi protons exhibit an apparent 1:2:1 distribution, complicated by the multiplicity of the 2H signal. We may attribute this behaviour to the same model as the 7·NO⁺ complex, where the oscillation process between two parallel opposite aromatic rings is slower on the NMR time scale; that is the picture we obtain from NMR reveals the NO⁺ guest to be next to one of the two rings, as presented in Figure 3 (7 is shown in the flattened-cone conformation, C*₂ᵥ* group of symmetry).

Finally, the distribution pattern of the signals in the ¹H NMR spectrum of the 9·NO⁺ complex seems to reveal the vicinity of the guest to the upper AB rings, attributable *inter alia* to the presence of the equatorial C-14 substituent. The higher shift of the involved protons may be related to the major proximity of the two aromatic rings, that is to a longer closeness of NO⁺ to the π-surface.

If these results are combined with those obtained in the UV-vis measurements, it can be concluded that one NO⁺ is encapsulated by interacting with just one of the four aromatic rings of the host.⁶

Addition of water or methanol to freshly NO⁺-exposed solutions of **1, 2, 7** and **9** in CHCl₃, resulted in complete complex dissociation and recovery of the corresponding free resorc[4]arenes. The same results were obtained when **9** and **11** were used as hosts (Table 2).

### Host-guest Dynamics

The possibility of observing the signals of both free and complexed species in the ¹H NMR spectrum is typical for the host-guest complexes with high Kₐₛₛ > 10⁶ M⁻¹ values. On the other hand, the NO⁺ guest, with a van der Waals diameter <2 Å,¹³ can migrate within the cavity at room temperatures. Complexes with cone resorc[4]arenes **1** and **7** showed a C*₂ₛ* symmetrical conformation, since only one aromatic ring interacts with NO⁺. Instead, the NMR spectrum of the free host **1** observed at room temperature exhibits a C*₄ᵥ* symmetry, indicating a fast exchange on the NMR time scale between two C*₂ᵥ* structures. The complex with 1,3-alternate resorc[4]arenes **9** and **11** should exhibit a C*₂ₕ* symmetry, with two different top and bottom halves of the skeleton. Instead, the apparent at room temperature symmetry is S₄, with equal top and bottom halves. At the same time, the complexation process is reversible, and the NO⁺ guest can still leave the resorc[4]arene cavity. Addition of water to freshly NO⁺-exposed solutions of **11** in CHCl₃ resulted in complete complex dissociation and recovery of the corresponding free resorcarene.

### Surface-linked resorc[4]arenes

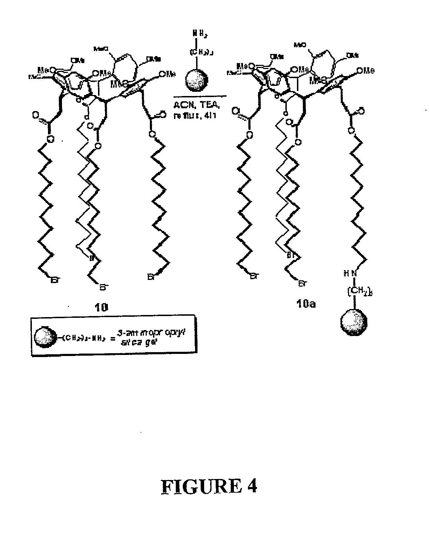

In these studies, spherical Daisogel SP-300-5P (5 µm particle size, 115 m²/g surface area) silica-gel from Daiso (Japan) was functionalized with 3-aminopropyltriethoxysilane to give the corresponding 3-aminopropylated silica-gel (elemental analysis: % C, 1.35; % H, 0.54; % N, 0.48; 375 µmoles g⁻¹. FTIR (DRIFT): 3432, 2978, 2935, 1874, 1630, 1095, 954, 802 cm⁻¹.) Both resorcarenes **7** and **10** readily reacted with the aminopropyl functionalized silica-gel, giving the immobilized systems **7a** and **10a** (see Figure 4 as an example), respectively. The surface-linking of the resorc[4]arene fragment on the modified silica-gel was confirmed by the appearance of intense v absorption bands at ~1870, 1616, 1507 and 1466 cm⁻¹ in the FTIR spectrum (KBr pellets).

Upon exposure to NO₂/N₂O₄, free resorc[4]arene 7 and immobilized macrocycle **7a** turned deep purple, both in solution (chlorinated solvents) and in the solid state. Conversely, **10** and **10a** (see below) turned deep yellow instead. In the NO₂ entrapment experiments, as a control, a stream of the gas was also passed through a Pasteur pipette loaded with the starting 3-aminopropylated silica-gel, and no colour appeared. The above colour change in the complex **7**·NO⁺ and **10**·NO⁺ indicates the occurrence of NO⁺ complexation. This once again emphasizes the role of resorc[4]arene cavities in the described processes and directly points at the use of complex **7**NO**+** and **10**NO⁺ for NO₂ detection and separation of other nitrogen oxides, especially NO, from NO₂.

### Conclusions

The signals in the ¹H NMR spectra of the NO⁺-complexes of resorcarenes **1, 2, 7, 9** and **11** definitely confirm the entrapment of the guest and its dependence on the conformation of the host. The signals (bold in Table 2) affected in the case of **9**·NO⁺ complex clearly fix the guest between the two A and B rings, which shift it on to each other.

The largely downfield values of the chemical shifts for the proton pairs 5/23 and 26/27, as compared with those of the other resorc[4]arenes, can be related to the shorter distance to be covered by the guest in its oscillation, that is to a longer time of interaction with the two equivalent faces of the same proton.

In the **2**·NO⁺ and **11**·NO⁺ complexes (from 1,3-alternate resorc[4]arenes) the cage is formed by the upper parts of B/D rings (He protons and neighbour methoxyl groups) and by the halves relative to Hi of the A/C rings. Probably concerted oscillations of the BD aromatic rings escort the transfer of the guest from each side to another. This may happen also in 1·NO⁺ and 7·NO⁺ complexes (from cone resorc[4]arenes), where the B/D rings interact with the guest and the flattened walls of A/C rings have the mere function of connecting the other two rings face to face. The difference between the ¹H NMR spectra of the two complexes may depend on faster oscillations of the B/D rings (and faster transfer of the guest) in 7·NO⁺; in the case of 1·NO⁺ the transfer process is slow on the NMR time scale and a series of partial pictures are registered in the resulting complex spectrum. The different oscillation rates can in turn be attributed to the presence of different substituents.

In summary, it is believed that one aromatic ring cannot interact with the nitrosonium ion (blank experiments), but two aromatic rings in a suitable arrangement are able to do so. When the guest is not yet completely released by one ring, it is already in the sphere of interaction of the other one.

### Smoking articles

Figures 5 and 6 illustrate examples of smoking articles according to the present invention. These illustrative smoking articles are in the form of cigarettes having a rod 1 of tobacco encased in a wrapper 2 attached to a smoke filter 3 by means of a tipping paper 4. For clarity, the tipping paper 4 is shown spaced from the wrapper 2, but in fact they will lie in close contact.

The absorbent of the present invention may be incorporated into the filter 3.

In figure 5, the smoke filter 3 comprises three cylindrical filter elements 3a, 3b, 3c. The first filter element 3a at the mouth end of the filter is 7mm in length, composed of cellulose acetate tow impregnated with 7% by weight of triacetin plasticiser having a 25mm water gauge pressure drop over its length. The second filter element 3b, positioned centrally is a cavity 5mm in length containing an absorbent according to the invention. The third filter element 3c adjacent the rod 1 is 15 mm in length, has a 90 mm water gauge pressure drop over its length, and comprises 80mg cellulose acetate tow. The tow is impregnated with 4% by weight of triacetin and has 80mg of granular activated carbon distributed evenly throughout its volume in a "Dalmatian" style.

The cigarette shown in Figure 6 is similar to that of Figure 5 except that the smoke filter 3 has four coaxial, cylindrical filter elements 3a, 3b, 3c and 3d. The first filter element 3a at the mouth end of the cigarette is 5mm in length, and composed of cellulose acetate tow impregnated with 7% by weight of triacetin plasticiser. The second filter element 3b, positioned adjacent the first filter element 3a is a cavity 5mm in length containing an absorbent according to the invention. The third filter element 3c adjacent the second filter element 3b is 10 mm in length and comprises cellulose acetate tow impregnated with 7% by weight of triacetin. The fourth filter element 3d lies between the third filter element 3c is 7mm in length and comprises 80mg of granular activated carbon. A ring of ventilation holes 5 is formed in the tipping paper 4 in a radial plane A-A which deliver air into the third filter element 3c about 3 mm downstream of the junction with the fourth filter element 3d when smoke is inhaled through the cigarette.

### Summary

The present invention relates to the use of a resorcin[4]arene to absorb one or more nitrogen oxides in a nitrogen oxide-containing medium.

We believe that the preferred resorcin[4]arenes may be represented by the resorc[4]arene of formula A 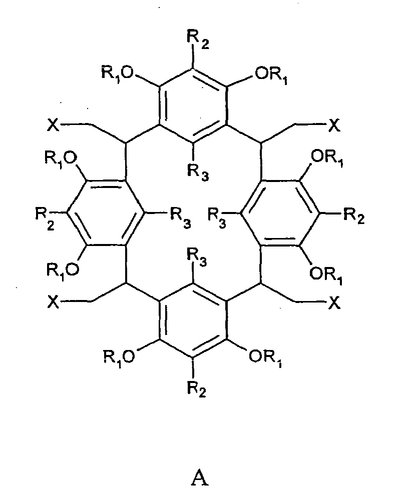 wherein
each of R₁, R₂, R₃, R₄, R₅, R₆, X and Y is independently H or a hydrocarbyl; and each n is independently 0 to 20.

The term "hydrocarbyl group" as used herein means a group comprising at least C and H and may optionally comprise one or more other suitable substituents. Examples of such substituents may include halo, alkoxy, nitro, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain hetero atoms. Suitable hetero atoms will be apparent to those skilled in the art and include, for instance, sulphur, nitrogen and oxygen. A nonlimiting example of a hydrocarbyl group is an acyl group.

A typical hydrocarbyl group is a hydrocarbon group. Here the term "hydrocarbon" means any one of an alkyl group, an alkenyl group, an alkynyl group, which groups may be linear, branched or cyclic, or an aryl group. The term hydrocarbon also includes those groups but wherein they have been optionally substituted. If the hydrocarbon is a branched structure having substituent(s) thereon, then the substitution may be on either the hydrocarbon backbone or on the branch; alternatively the substitutions may be on the hydrocarbon backbone and on the branch.

In some aspects of the present invention, one or more hydrocarbyl groups is independently selected from optionally substituted alkyl group, optionally substituted haloalkyl group, aryl group, alkylaryl group, alkylarylalkyl group, and an alkene group.

In some aspects of the present invention, one or more hydrocarbyl groups is independently selected from C₁-C₁₀ alkyl group, such as C₁-C₆ alkyl group, and C₁-C₃ alkyl group. Typical alkyl groups include C₁ alkyl, C₂ alkyl, C₃ alkyl, C₄ alkyl, C₅ alkyl, C₇ alkyl, and C₈ alkyl.

For some aspects, the hydrocarbyl group may comprise a carbonyl group. The carbonyl group may have the formula COOH.

In some aspects of the present invention, one or more hydrocarbyl groups may be independently selected from one or more oxyhydrocarbyl groups. The term "oxyhydrocarbyl" group as used herein means a group comprising at least C, H and O and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the oxyhydrocarbyl group comprises more than one C then those carbons need not necessarily be linked to each other. For example, at least two of the carbons may be linked via a suitable element or group. Thus, the oxyhydrocarbyl group may contain hetero atoms. Suitable hetero atoms will be apparent to those skilled in the art and include, for instance, sulphur and nitrogen.

In one embodiment of the present invention, the oxyhydrocarbyl group is a oxyhydrocarbon group. Here the term "oxyhydrocarbon" means any one of an alkoxy group, an oxyalkenyl group, an oxyalkynyl group, which groups may be linear, branched or cyclic, or an oxyaryl group. The term oxyhydrocarbon also includes those groups but wherein they have been optionally substituted. If the oxyhydrocarbon is a branched structure having substituent(s) thereon, then the substitution may be on either the hydrocarbon backbone or on the branch; alternatively the substitutions may be on the hydrocarbon backbone and on the branch.

Some of the compounds of the present invention may exist as stereoisomers and/or geometric isomers ― e.g. they may possess one or more asymmetric and/or geometric centres and so may exist in two or more stereoisomeric and/or geometric forms. The present invention contemplates the use of all of the individual stereoisomers and geometric isomers of those compounds, and mixtures thereof.

The present invention also relates to relates to nitrosonium complexes of such resorcin[4]arenes.

Various modifications and variations of the described aspects of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the scope of the following claims.

### REFERENCES

(1) (a) Lerdau, M. T.; Munger, J. W.; Jacob, D. J. Science 2000, 5488, 2291-2293.
(2) Kirsch, M.; Korth, H.-G.; Sustmann, R.; de Groot, H. Biol. Chem. 2002, 383, 389-399.
(3) (a) Asfari, Z.; Böhmer, V.; Harrowfield, J.; Vicens, J., Eds. Calixarene 2001; Kluwer Academic: Dordrecht, 2001. (b) Gutsche, C. D. Calixarenes; Royal Society of Chemistry: Cambridge, 1998. (c) Rudkevich, D. M. Bull. Chem. Soc. Jpn. 2002, 75, 393-413.
(4) (a) Arduini, A.; Fabbi, M.; Mirone, L.; Pochini, A.; Secchi, A.; Ungaro, R. J. Org. Chem. 1995, 60, 1454-1458. (b) Conner, M.; Janout, V.; Regen, S. L. J. Am. Chem. Soc. 1991, 113, 9670-9671. (c) Scheerder, J.; Vreekamp, R. H.; Engbersen, J. F. J.; Verboom, W.; van Duynhoven, J. P. M.; Reinhoudt, D. N. J. Org. Chem. 1996, 61, 3476-3481.
(5) (a) Ikeda, A.; Shinkai, S. J. Am. Chem. Soc. 1994, 116, 3102-3110. (b) Ikeda, A.; Tsuzuki, H.; Shinkai, S. Tetrahedron Lett. 1994, 35, 8417-8420. (c) Ikeda, A.; Shinkai, S. J. Chem. Soc., Chem. Commun. 1994, 2375-2376. (d) Ikeda, A.; Tsudera, T.; Shinkai, S. J. Org. Chem. 1997, 62, 3568-3574.
(6) Rathore, R.; Lindeman, S. V.; Rao, K. S. S.; Sun, D.; Kochi, J. K. Angew. Chem., Int. Ed. 2000,39,2123-2127.
(7) Addison, C. C. Chem. ReV. 1980,80,21-39.
(8) (a) Bosch, E.; Kochi, J. K. J. Org. Chem. 1994, 59, 3314-3325 and references cited therein. (b) Evans, J. C.; Rinn, H. W.; Kuhn, S. J.; Olah, G. Inorg. Chem. 1964,3,857-861.
(9) Zyryanov, G. V.; Kang, Y.; Rudkevich, D. M. J. Am. Chem. Soc. 2003, 125, 2997-3007.
(10) Botta, B.; Di Giovanni, M. C.; Delle Monache, G.; De Rosa M. C.; Gács-Baitz, E.; Botta, M.; Corelli, F.; Tafi, A.; Santini, A.; Benedetti, E.; Pedone, C.; Misiti, D. J. Org. Chem. 1994,59,1532-1541
(11) Botta, B.; Delle Monache, G.; de Rosa, M., C.; Seri, C.; Benedetti, E.; Iacovino, R.; Botta, M.; Corelli, F.; Tafi, A.; Malignai, V.; Gács-Baitz, E.; Santini, A.; Misiti, D. J. Org. Chem. 1997,62,1788-1794.
(12) Borodkin, G. I.; Shubin, V. G. Russ. Chem. ReV. 2001, 70, 211-230.
(13) Rosokha, S. V.; Kochi, J. K. J. Am. Chem. Soc. 2001, 123, 8985-8999.

## Claims

1. Use of a resorcin[4]arene to absorb one or more nitrogen oxides in a nitrogen oxide-containing medium.

2. Use according to claim 1 wherein the one or more resorc[4]arene is of formula I 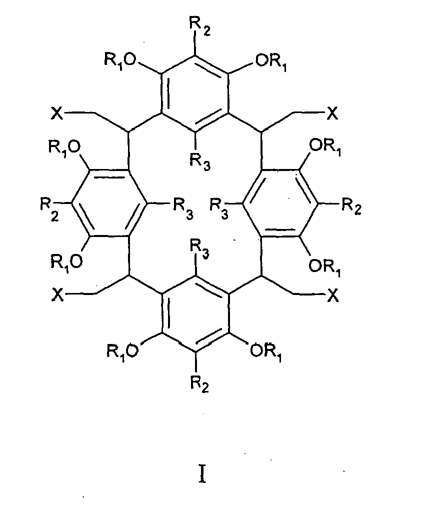 wherein
each R₂ and R₃ is independently H, alkoxy, alkyl, aryl, aralkyl, halogen, cyano, nitro or carboxylate;
each X is independently COOR₄ or CH₂-halogen;
each R₄ is independently H, alkyl, aryl, aralkyl or -(CH₂)ₙCH₂Y;
each n is independently 0 to 20;
each Y is independently CH₂-halogen or CH=CR₅R₆;
each R₁, R₅ and R₆ is independently H, alkyl, aryl or aralkyl; . wherein said alkyl, aryl and aralkyl groups may be optionally substituted by one or more substituents selected from halogen, OH, alkoxy, NO₂, NH₂, N(alkyl)₂, C(halo)₃, carboxylate and cyano.

3. Use according to claim 2 wherein the halogen is Cl, Br, or I.

4. Use according to claim 2 or claim 3 wherein each R₂ and R₃ are H.

5. Use according to any one of claims 2 to 4 wherein each R₁, R₅ and R₆ is independently H or alkyl.

6. Use according to any one of claims 2 to 5 wherein each R₄ is independently H, alkyl, or -(CH₂)ₙCH₂Y;

7. Use according to any one of claims 2 to 6 wherein each X is the same.

8. Use according to claims 2 to 7 wherein each R₁ is the same.

9. Use according to claim 8 wherein R₁ is alkyl.

10. Use according to any one of claims 2 to 9 wherein
X is COOR₄ or CH₂Br;
R₄ is H, Me, Et, ⁱPr, or -(CH₂)ₙCH₂Y;
n is 2 to 15;
Y is CH₂Br or CH=CH₂; and R₁ is Me.

11. Use according to any one of claims 2 to 10 wherein n is 2 to 12.

12. Use according to any one of claims 2 to 11 wherein
X is COOR₄ or CH₂Br; and
R₄ is H, Me, Et, ⁱPr or -(CH₂)ₙCH₂Br;
n is 2 to 11; and
R₁ is Me.

13. Use according to any one of claims 2 to 11 wherein
X is COOR₄ or CH₂Br; and
R₄ is H, Me, Et, ⁱPr, or -(CH₂)ₙCH=CH₂;
n is 2 to 9; and
R₁ is Me.

14. Use according to any one of claims 2 to 11 wherein
X is COOR₄ or CH₂Br; and
R₄ is H, Me, Et, ⁱPr, -(CH₂)₁₁CH₂Br or -(CH₂)₉CH=CH₂; and
R₁ is Me.

15. Use according to any preceding claim wherein the nitrogen oxide is NO₂ and/or N₂O₄.

16. Use according to any preceding claim wherein the resorc[4]arene is tethered to a support material.

17. Use according to any preceding claim wherein the support material is a modified silica gel.

18. Use according to any preceding claim wherein the support material is aminopropylated silica gel.

19. Use according to claim 18 wherein the resorc[4]arene is linked to the aminopropylated silica gel via a bond formed by reacting the amino group of aminopropylated silica gel with a resorc[4]arene of formula Ia, 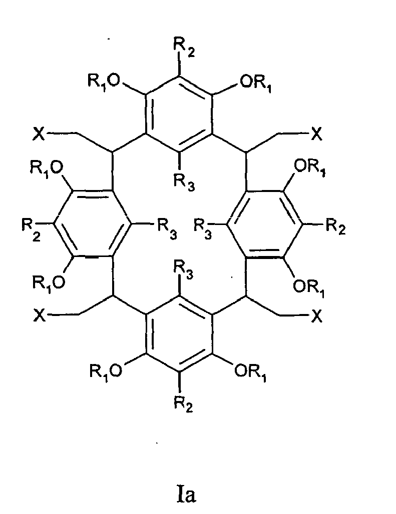 wherein
each R₂ and R₃ is independently H, alkoxy, alkyl, aryl, aralkyl, halogen, cyano, nitro or carboxylate;
each X is independently COOR₄ and each R₄ is -(CH₂)ₙCH₂Y;
each n is independently 0 to 20;
each Y is independently CH₂-halogen;
each R₁ is independently H, alkyl, aryl or aralkyl; wherein said alkyl, aryl and aralkyl groups may be optionally substituted by one or more substituents selected from halogen, OH, alkoxy, NO₂, NH₂, N(alkyl)₂ (preferably NMe₂), C(halo)₃ (preferably CF₃), carboxylate and cyano.

20. Use according to claim 19 wherein for said compound of formula Ia,
X is COOR₄ and R₄ is -(CH₂)ₙCH₂Y;
n is 0 to 20;
Y is CH₂-halogen, wherein halogen is Cl, Br, or I;
R₁ is alkyl; and
R₂ and R₃ are H;
R₁ is alkyl.

21. Use according to claim 20 wherein the halogen is Br.

22. Use according to any preceding claim wherein the gaseous mixture is produced by burning tobacco.

23. Use according to any preceding claim which absorbs substantially all the NO₂ from the gaseous mixture.

24. Use according to any preceding claim which selectively absorbs NO₂ from the gaseous mixture.

25. A method of forming a nitrosonium complex, said method comprising contacting a resorc[4]arene with a nitrogen oxide.

26. A nitrosonium complex of formula II 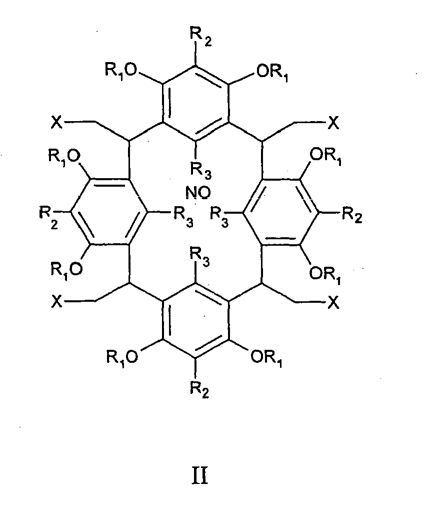 wherein R₁, R₂, R₃ and X are as defined in any one of claims 2 to 7.

27. A complex formed by contacting a resorc[4]arene with a nitrogen oxide.

28. Use of a complex according to claim 26 or claim 27 as a nitrosating reagent.

29. Use of a complex according to claim 26 or claim 27 as an NO⁺ transfer agent.

30. A method of entrapping one or more nitrogen oxides, said method comprising contacting one or more resorc[4]arenes with a gaseous mixture comprising one or more nitrogen oxides.

31. A method of absorbing one or more nitrogen oxides from tobacco smoke, said method comprising filtering the tobacco smoke through a filter element comprising one or more resorc[4]arenes.

32. A method of selectively absorbing nitrogen oxide-containing combustion products from tobacco smoke, said method comprising allowing tobacco smoke to come into contact with a filter element comprising one or more resorc[4]arenes.

33. A filter element for absorbing one or more nitrogen oxides in a gaseous mixture, said filter element comprising one or more resorc[4]arenes and optionally one or more additional filter materials.

34. A filter element according to claim 26 which is for use with a smoking article.

35. A filter element for selectively absorbing NO₂-containing combustion products produced by burning a smoking article, said filter element comprising as filter material one or more resorc[4]arenes.

36. A smoking article comprising a filter element according to any one of claims 33 to 35.

37. A smoking article according to claim 36 which is a cigarette, cigar, cigar holder or pipe.

38. A filtration apparatus for absorbing, or reducing the level of, nitrogen oxides in a gaseous mixture, said apparatus comprising:
a chamber for receiving the gaseous mixture;
inlet and/or outlet means;
means for absorbing, or reducing the level of, one or more nitrogen oxides, said
means comprising one or more resorc[4]arenes; and
optionally a fluid transfer means for circulating the gaseous mixture around the chamber.

39. A filtration apparatus according to claim 38 wherein the fluid transfer means is a pump.

40. A filtration apparatus according to claim 38 or 39 wherein the chamber is a closed system.

41. A method according to any one of claims 30 to 32, or a filter element according to any one of claims 33 to 35, or a filtration apparatus according to any one of claims 38 to 40, wherein the resorc[4]arene is as defined in any one of claims 2 to 14 or 16 to 21.

42. A smoke filter or a wrapper or a component of a smoking article comprising one or more resorc[4]arenes and optionally one or more additional filter materials.

43. A method, use, nitrosonium complex, filter element, filtration apparatus, or smoking article substantially as described herein with reference to the accompanying figures.
